# EUROPEAN PATENT SPECIFICATION

(11) **EP 1 871 395 B1**
(45) Date of publication and mention of the grant of the patent: **26.10.2011**
(21) Application number: 06721748.9
(22) Date of filing: 31.03.2006
(51) Int. Cl.: A61K 36/8962, A61K 31/722, A61P 31/10

(54) **GARLIC EXTRACT AND CHITOSAN COMPOSITIONS, AND USES THEREOF**
KNOBLAUCHEXTRAKT UND CHITOSAN-ZUSAMMENSETZUNGEN UND IHRE VERWENDUNGEN
EXTRAIT D'AIL ET COMPOSITIONS DE CHITOSANE ET LEURS UTILISATIONS

(30) Priority: 01.04.2005 US 667096 P
(43) Date of publication of application: 02.01.2008
(73) Proprietor: Université Laval, Québec, QC G1K 7P4 (CA)
(72) Inventor: BENHAMOU, Nicole, Québec, Québec G1V 4T8 (CA); COUTURE, Sébastien, Saint-Nicolas, Québec G7A 3Y7 (CA)
(74) Representative: Chevalier, Renaud Philippe
(86) International application number: PCT/CA2006/000491
(87) International publication number: WO 2006/102766

(56) References cited:
- JP-A- 09 132 510
- JP-A- 10 175 878
- PRANOTO Y ET AL: "Enhancing antimicrobial activity of chitosan films by incorporating garlic oil, potassium sorbate and nisin" LEBENSMITTEL WISSENSCHAFT UND TECHNOLOGIE, 24 November 2004 (2004-11-24), XP002533048 Retrieved from the Internet: URL:doi:10.1016/j.lwt.2004.09.014> [retrieved on 2009-06-18] -& PRANOTO Y ET AL: "Enhancing antimicrobial activity of chitosan films by incorporating garlic oil, potassium sorbate and nisin" LEBENSMITTEL WISSENSCHAFT UND TECHNOLOGIE, ACADEMIC PRESS, LONDON, GB, vol. 38, no. 8, 1 December 2005 (2005-12-01), pages 859-865, XP004951627 ISSN: 0023-6438
- MAZUR STANISLAW ET AL: "Effect of some compounds on the decay of strawberry fruits caused by Botrytis cinerea Pers" MEDEDELINGEN FACULTEIT LANDBOUWKUNDIGE EN TOEGEPASTE BIOLOGISCHE WETENSCHAPPEN UNIVERSITEIT GENT, vol. 66, no. 2A, 2001, pages 227-231, XP008107504 ISSN: 1373-7503
- MAZUR S. ET AL.: 'Effect of Garlic Extract and Chitosan Water Solution on Botrytis cinerea Pers' BULLETIN OF THE POLISH ACADEMY OF SCIENCES BIOLOGICAL SCIENCES vol. 47, no. 2-4, 1999, pages 113 - 118, XP008107465
- MUSHIN T.M. ET AL.: 'Effect of garlic bulb extract on the growth and enzymatic activities of rhizosphere and rhizoplane fungi' MYCOPATHOLOGIA vol. 152, 2000, pages 143 - 146, XP019259624
- TANSEY M.R. AND APPLETON J.A.: 'Inhibition of fungal growth by garlic extract' MYCOLOGIA vol. 67, 1975, pages 409 - 413, XP008121807
- BEN-SHALOM N. ET AL.: 'Controlling gray mould caused by Botrytis cinerea in cucumber plants by means of chitosan' CROP PROTECTION vol. 22, 2003, pages 285 - 290, XP008121745
- RABEA E.I. ET AL.: 'Chitosan as Antimicrobial Agent: Applications and Mode of Action' BIOMACROMOLECULES vol. 4, no. 6, 2003, pages 1457 - 1465, XP002339032

## Description

### TECHNICAL FIELD

The present invention relates to improvements in the field of garlic extract compositions. In particular, this invention relates to antifungal compositions comprising a garlic extract that can be used to treat a plant, a vegetable plant, a fruit plant or a flower (ornamental) plant as well as products thereof such as seeds, leaves, roots, vegetable, fruit or flower.

### BACKGROUND OF THE INVENTION

The treatment of plants or products thereof before or after harvest is very important in order to obtain plants, flowers, vegetables or fruits of the highest quality. For many years, these treatments have been based on the use of chemical products. This market is currently dominated by chemical-based products. Considering the increasing inputs of toxic substances in the environment and the food chain, the development of a cost-competitive biological control approach to reduce or even eliminate pesticide use is an option that is urgently needed. Such a strategy may generate major economic benefits by enabling growers to manage powdery mildew disease and prevent high economic loss, without relying solely on chemically-based pesticides. Evidence has been provided that natural or biological products could become an appreciable alternative to chemicals, not only in terms of environmental protection, but also in terms of human health.

More specifically, the management of powdery mildew disease has become one of the most challenging research area in plant pathology (Bélanger et al. Plant-Microbe Interaction and Biological Control., 1998, NY, U.S.A.: Marcel Decker, pp. 89-109.). In the past two decades, various measures have been recommended for controlling these diseases, including disinfections of greenhouse structures and cultivation of resistant cultivars. However, the wide host range of powdery mildew pathogens as well as their ability to proliferate abundantly under the environmental conditions that usually prevail in greenhouses have markedly reduced the potential of such methods as management strategies. Chemicals such as sulfur, Dinocap™, Benomyl™, as well as systemic fungicides have been widely applied but have not always proved totally effective, at least at concentrations under their phytototoxic levels (Menzies et al, Can. J. Plant Pathol., 1996, 18: 186-193). In spite of much effort, powdery mildew disease continue to be a major problem for producers worldwide and there is now a strong and irreversible impetus to use and commercialize biological control approaches as reliable substitutes of chemically-based pesticides.

Garlic or garlic extracts are known to contain an array of substances with beneficial health-related biological properties (Agarwal, K.C., 1996. Therapeutic actions of garlic constituents. Med. Res. Rev. 16: 111-124). Moreover, Lemar et al.. (J. Appl. Microbiol, 2002, 93 : 398-405) recently reported that garlic extracts displayed a growth-inhibiting activity against *Candida albicans,* a fungus multiplying in the oral-gastrointestinal tract of humans. This document indicated also that fresh garlic extracts had a greater efficacy than dehydrated extracts.

In contrast to the increasing amount of data related to the garlic-human health relationship, little scientific information is available regarding the potential use of garlic extracts for crop protection. Recent observations revealed that garlic extracts displayed the ability to markedly reduce the growth of some phytopathogenic fungi *in vitro* (Bianchi et al. Plant Dis., 1997, 81: 1241-1246.). The mechanisms by which garlic may contribute to biological control of plant pests (including pathogens, insects, and nematodes) are largely unknown although a number of hypotheses including toxic activity driven by the action of allicin on free thiol groups of microbial enzymes have been raised. While there is no doubt that direct effects of garlic compounds on the pathogen populations should be responsible, at least partly, for an enhanced plant protection, the possibility that garlic may also trigger indirect effects by sensitizing the plant to defend itself through the activation of defense genes has not been investigated.

It has been shown that garlic extracts are highly sensitive to environmental factors such as light, the pH of the solution containing the garlic extract, oxidation, and even contamination by microorganisms (Hong et al. Sci. Food Agric, 2001, 81: 397-403). Indeed, aqueous garlic extracts have been found to be highly unstable and to undergo rapid discoloration at room temperature and even at 4 and -20° C. Browning and greening of aqueous garlic solutions are among the main problems responsible for a marked decrease in the biological activity of garlic components. Such instability phenomena are thought to be due to the oxidation of phenolic compounds. The shelf-life of aqueous compositions comprising fresh garlic extracts is thus substantially short. Moreover, it may also be seen as a tedious task for a user to prepare, before each application or treatment, fresh solutions of garlic extracts. In addition, when a user needs to prepare a considerable amount of garlic solution to be applied on several plants over a few hours, the quality and effectiveness of the solution may vary between the first and the last plants treated.

U.S. Pat. No. 6,231,865 describes that the composition of a garlic extract with an essential oil (such as cotton seed oil, cinnamon oil, or mineral oil) in the presence of sodium dodecyl sulfate, can reduce the severity of powdery mildew disease in cucumber and rose plants. However, such compositions may require more than an application per week to provide good control of powdery mildew disease. In addition, a major problem with these compositions concerns the presence of a chemical product, sodium dodecyl sulfate, which is phytotoxic.

Mazur et el. (Med. Fac. Landbouww.Univ. Gent, 2001, 66/2A:227-231) show the results of field trials in which the effect of either garlic extract or chitosan is tested against *Botrytis cinerea.*

In light of the above, the development of a composition which could display a biological activity superior to that of an aqueous composition of garlic extract alone is a desirable outcome. In parallel, obtaining a stable composition in which garlic components are preserved from environmental factors is another essential outcome if one wants to develop an efficient and reliable biofungicide.

### SUMMARY OF THE INVENTION

The present invention provides a method for inhibiting the growth of a fungus in plant. The method comprises the step of contacting the antifungal composition described herein with the plant.

In a first aspect, the antifungal composition comprises a garlic extract and a chitosan or a salt thereof. In an embodiment, the antifungal composition may have a pH ranging from 5.0 to 6.0, and in a further embodiment, from 5.2 to 5.8. In a further embodiment, the chitosan salt is selected from the group consisting of chitosan lactate, chitosan propionate, chitosan sorbate and chitosan gluconate. In another embodiment, the chitosan salt is chitosan lactate.

In still another embodiment, the chitosan is native chitosan. In still another embodiment, the composition further comprises a vegetable oil. In a further embodiment, the antifungal composition is an aqueous solution. In still a further embodiment, the concentration of the garlic extract in the composition is ranging from about 5 mg/mL to 95 mg/mL, in still a further embodiment, from about 10 mg/mL to 80 mg/mL, in still a further embodiment, from about 20 mg/mL to 70 mg/mL. In yet another embodiment, the concentration of the chitosan or salt thereof in the composition is ranging from about 0.1 mg/mL to 6 mg/mL, in yet another embodiment, from about 0.2 mg/mL to 5 mg/mL, in still another embodiment from about 0.5 mg/mL to 4 mg/mL. In still another embodiment, the antifungal composition is capable of inhibiting the growth of a pathogen. In an embodiment, the pathogen is a fungus, and, in a further embodiment, the pathogen is selected from the group consisting of *Botrytis cinerea, Fusarium oxysporum, Penicillium digitatum, Phytophthora megasperma, Pythium aphanidermatum, Pythium ultimum, Rhizoctonia solani, Sclerotinia sclerotiorum, Fusarium sambucinum, Fusarium graminearum, Verticillium dahliae,* a powdery mildew pathogen and a rust pathogen. In yet another embodiment, the growth of the pathogen occurs in a plant and in still another embodiment, the plant is selected from the group consisting of a vegetable plant, a fruit plant and an ornamental plant. In yet another embodiment, the growth of the pathogen occurs in a product of a plant, and in still another embodiment the product of said plant is selected from the group consisting of a vegetable and a fruit. In yet another embodiment, the antifungal composition is capable of inhibiting at least 50% of the growth of said pathogen in a plant or a product of the plant and in yet a further embodiment is capable of inhibiting at least 90% of the growth of said pathogen in a plant or a product of the plant. In still another embodiment, the antifungal composition is capable of controlling powdery mildew disease in a plant or a product of the plant. Various embodiments of the plant and its product have been described above. In an embodiment, the ability of the antifungal composition to inhibit the growth of the pathogen or to control powdery mildew disease is being preserved in storage for a period of over 260 days. In an embodiment, the antifungal composition is in powder form. In another embodiment, the garlic extract is a dehydrated garlic extract. In yet another embodiment, when the antifungal composition is in powder form, it comprises between about 50% to 99% by weight of said garlic extract and/or comprises between about 5% to 20% by weight of the chitosan or salt thereof.

Various embodiments of the pathogen have been described above. In an embodiment, the antifungal composition is contacted with a leaf and/or a foliage of the plant. In another embodiment, the antifungal composition is contacted with said plant at least once in a period of 5 days, in a further embodiment, at least once in a period of 7 days, in yet a further embodiment, at least once in a period of 14 days, and in still a further embodiment, at least once in a period of 20 days.

According to still another aspect, the present invention provides a method for controlling powdery mildew disease in a plant. The method comprises the steps of contacting the antifungal composition described herein with the plant. In an embodiment, the antifungal composition is contacted with a leaf and/or a foliage of the plant. In another embodiment, the antifungal composition is contacted with said plant at least once in a period of 5 days, in a further embodiment, at least once in a period of 7 days, in yet a further embodiment, at least once in a period of 14 days, and in still a further embodiment, at least once in a period of 20 days.

In another aspect, the present invention provides a method for preparing an antifungal composition. The method comprises the steps of mixing a garlic extract with a chitosan or a salt thereof, thereby obtaining the antifungal composition. In an embodiment, the garlic extract is a dehydrated garlic extract and/or the garlic extract is in powder form. In another embodiment, the chitosan or salt thereof is in powder form. In another embodiment, the composition is an aqueous solution. In a further embodiment, the method further comprises the step of adjusting the pH of said antifungal composition between 5.0 and 6.0, and in a further embodiment, between 5.2 and 6.8.

### BRIEF DESCRIPTION OF THE FIGURES

Fig. 1 shows (A) a cucumber leaf infected with powdery mildew, (B) the same cucumber leaf that has been treated with the composition 1 described herein, (C) a scanning electron micrograph of the infected cucumber leaf shown in A, showing turgescent hyphae and conidia at the leaf surface, and (D) a scanning electron micrograph of the treated cucumber leaf of B, showing collapsed and damaged hyphae.
Fig. 2 shows (A) a tomato leaf infected with powdery mildew, and (B) a tomato leaf treated with the composition 1 described herein.
Fig. 3 shows (A) strawberry leaves infected with powdery mildew, (B) the same strawberry leaves that have been treated with the composition 1 described herein, (C) a scanning electron micrograph of the surface of one of the strawberry leaves shown in A, wherein the spores are intact, and (D) a scanning electron micrograph of the surface of one of the strawberry leaves shown in B, wherein spores are highly collapsed.
Fig. 4 shows the effect of the composition described herein on rose powdery mildew disease as compared to that of the fungicide MELTATOX™.
Fig. 5 shows the effect of the composition described herein on peony powdery mildew disease as compared to that of the fungicide NOVA™.
Fig. 6 shows the effect of the composition described herein as compared to that of MILSTOP™, FONGINEEM™, and SILIFORCE™ on the control of powdery mildew on cucumber plants.

### DETAILED DESCRIPTION OF PREFERRED EMBODIMENTS

It is therefore disclosed composition comprising a garlic extract and having an enhanced antifungal activity as compared to that of a composition consisting of a garlic extract and wate and a composition comprising a garlic extract which preserves the activity or properties of the garlic extract over a prolonged period of time. It is also disclosed a composition comprising a garlic extract and having an extended shelf life as compared to that of a composition consisting of a garlic extract and water. It is also disclosed a composition comprising a garlic extract and being disclosed efficient for controlling powdery mildew disease. It is also disclosed a composition that is biodegradable, compatible with current cultural practices, and that is not phytotoxic.

The compositions described herein comprise a garlic extract. As used herein, the term "garlic extract" is intended to mean a processed garlic product. The garlic *(Allium sativum)* that is being processed can be fresh, frozen or dehydrated. The garlic extract can be processed in various ways, it can be cut, crushed, pulverized, dried, frozen. The garlic extract may also be processed in a powder form. The garlic extract can contain all the components present in a non-processed garlic or it can contain only certain components of the non-process garlic (peeled vs. unpeeled garlic cloves for example). The garlic extract can also be a crude extract (a garlic extract containing the majority of the components of a non-processed garlic).

The compositions described herein also comprise chitosan or a salt thereof. Chitosan is the N-deacetylated product of chitin (e.g. polysaccharide analogous in chemical structure to cellulose except that the repeating unit is a (1,4)-linked N-acetyl-D-glucosamine, a compound consisting of glucose derivative units joined to form a long, unbranched chain). Chitosan is present in the exoskeletons of many arthropods, invertebrate organisms and the cell walls of most fungi. Large quantities of chitosan can also be obtained from the chitin of crustacean shell waste. The presence of chitosan or its salt in the composition increases the antifungal activity of the composition and extends the shelf life of the composition. In an embodiment, the chitosan present in the composition is in the form of a salt. The chitosan salt can be, for example, selected from the group consisting of chitosan lactate, chitosan propionate, chitosan sorbate and chitosan gluconate. The chitosan salt is preferably chitosan lactate. The use of a chitosan salt in the composition may be advantageous, especially if the composition is an aqueous solution, because chitosan salts are water-soluble. The chitosan present in the solution can also be native chitosan. If native chitosan is used, it is preferably treated with an acid prior to its addition to the composition to render it more water-soluble.

In an embodiment, the pH of the composition can be adjusted to optimize its antifungal activity (e.g. longer-lasting activity, more potent activity and/or extended shelf life). The pH of the composition can be, for example, ranging from 5.0 to 6.0 and, preferably from 5.2 to 5.8.

The compositions described herein can also contain an emulsifier. The role of the emulsifier in the composition is to stabilize the solubilized particles of the composition, therefore increasing the shelf-life of the composition.

In an embodiment, the compositions described herein can also contain an oil, and in a further embodiment, a vegetable oil. The role of the vegetable oil in the composition is to homogenize the compositions.

The compositions described herein can be in a liquid form or in a solid form. When the compositions are in a liquid form, it may be a water-based solution (e.g. an aqueous solution). When the compositions are in a liquid form, the concentration of the garlic extract should be adjusted in order to achieve desired antifungal properties. In an embodiment, the concentration of the garlic extract in the compositions may be ranging from about 5 mg/mL to 95 mg/mL, from about 10 mg/mL to 80 mg/mL or from about 20 mg/mL to 70 mg/mL. The concentration of the chitosan or salt thereof in the liquid composition should also be adjusted to achieve desired antifungal properties. In an embodiment, the concentration of the chitosan or its salt in the composition may be less than 6 mg/mL or may be ranging from about 0.1 mg/mL to 6 mg/mL, from about 0.2 mg/mL to 5 mg/mL or from about 0.5 mg/mL to 4 mg/mL. When the composition is in a solid form, the composition may be in a powder form. When the compositions are in powder form, the compositions can comprise at least 50% by weight of the garlic extract in powder form, more preferably at least 80% by weight of the garlic extract in powder form. The compositions can also comprise between about 50 to 99% by weight of the garlic extract. More preferably, the compositions can also comprise between about 80 to 91% by weight of the garlic extract The garlic extract is preferably a dehydrated garlic extract. The compositions in solid form can also comprise at least 5% by weight of chitosan or salt thereof. The compositions can also comprise at least 9% by weight of chitosan or salt thereof. Alternatively, the compositions can comprise between about 5 to 20% by weight of the chitosan or salt thereof, and more preferably between about 9 to 20% by weight of the chitosan or salt thereof. The compositions in powder form can be sealed in a container under vacuum. The container can be a polymeric material pouch package. Once rehydrated, the compositions in powder form exhibit antifungal activity.

The term "plant" as used herein refers to a plant, a plant tissue, a part of a plant, a plant organ or a plant product. A plant tissue can be any or every tissue in a plant, for example epidermal tissue, cortical tissue, vascular tissue, etc. A part of the plant may be, for example, foliage, a flower, a leaf, a root, a stem, etc. A plant product may be, for example, a seed, a vegetable, a fruit, etc.

As used herein, the term "antifungal" is intended to mean the property of the composition to impede, halt or slow down the growth of a microorganism or a pathogenic microorganism (also referred to as a pathogens), being fungal plant pathogens. The combination of the garlic extract and chitosan (or salt thereof) has an impressive antifungal synergistic effect. The combination of the garlic extract and the chitosan or salt thereof can be a more effective antifungal agent than if an equivalent amount of the garlic extract or the chitosan or salt thereof was used alone in aqueous solution. Moreover, the composition of the garlic extract and the chitosan or salt thereof can also be more effective for controlling powdery mildew than if an equivalent amount of the garlic extract or the chitosan or salt thereof was used alone in aqueous solution.

The compositions of the present invention can be used for treating a plant, a vegetable plant or vegetable thereof, or a fruit plant or fruit thereof. Such use is preferably made in a greenhouse, a garden, or a field. The compositions can also be used for treating grass or for treating plants in a garden or a golf course.

The antifungal compositions described herein are capable of inhibiting the growth of a fungal plant pathogen. As used herein, the term "pathogen" is intended to mean a microorganism that can cause a disease in a subject (such as a plant or a product of a plant). In an embodiment, the pathogen is a fungus and, in a further embodiment, a fungus capable of infecting a plant or causing a disease in a plant. Pathogens may be, for example, from the *Botrytis* sp. (e.g. *Botrytis cinerea),* from the *Fusarium* sp. (e.g *Fusarium oxysporum, Fusarium sambucinum, Fusarium graminearum),* from *the Penicillium* sp. (e.g. *Penicillium digitatum),* from the *Phytophthora* sp. (e.g. *Phytophthora megasperma),* from the *Pythium* sp. (e.g. *Pythium aphanidermatum, Pythium ultimum),* from the *Rhizoctonia* sp. (e.g. *Rhizoctonia solani),* from the *Sclerotinia* sp. (e.g. *Sclerotinia sclerotiorum),* from the *Verticillium* sp. (e.g. *Verticillium dahliae),* a powdery mildew pathogen or a rust pathogen.

The expression "growth of a pathogen" as used herein refers to the life cycle of a pathogen including, but not limited to, development, replication and reproduction (e.g. sporulation). The expressions "inhibition of the growth of a pathogen" and "growth inhibition" as used herein refer to the disruption of the life cycle of the pathogen, on any or every level of the life cycle, in such a way that it results in controlling, reducing, slowing-down or halting of the life cycle of the pathogen. A disruption of the regular life cycle of the pathogen leading to the death of the pathogen is also encompassed within the expressions "inhibition of the growth of a pathogen" an "growth inhibition" as used herein.

As mentioned above, the pathogen inhibited by the antifungal composition can grow in a plant tissue, thereby causing a disease in a plant or part of a plant. The plant may be a vegetable plant (such as pepper plant, lettuce plant, eggplant plant or a zucchini plant), a fruit plant (such as a strawberry plant, a raspberry plant, a blueberry plant, a grape plant, a melon plant, a cucumber plant, a tomato plant or an apple tree), an ornamental plant (such as a rose, a poinsettia, or a peony), a shrub or grass. As mentioned above, the pathogen inhibited by the antifungal composition can grow on the product of a plant, such as a seed, a vegetable (such as a pepper, a lettuce, an eggplant or a zucchini) or a fruit (such as a strawberry, a raspberry, a blueberry, a grape, a melon, a cucumber, a tomato or an apple).

The antifungal composition is also capable of controlling powdery mildew disease. Powdery mildew are caused by obligate parasites of the *Erysiphaceae* family. Powdery mildew affect plants (such as grass) or parts thereof, more specifically vegetable plants (such as a pepper plant, a lettuce plant, an eggplant plant or a zucchini plant), fruit plants (such as a strawberry plant, a raspberry plant, a blueberry plant and a grape plant), ornamental plants (such as a rose, a poinsettia or a peony) or parts thereof (such as a flower, a leaf, a root, a stem, etc). Powdery mildew disease can also affect products of a plant, such as a seed, a vegetable (such as a pepper, a lettuce, an eggplant or a zucchini) or a fruit (such as a strawberry, a raspberry, a blueberry and a grape).

The antifungal composition described herein is a stable product. Tests have shown that the compositions retain their capacity to inhibit the growth of plant pathogen (such as a fungal plant pathogen) or to control of powdery mildew disease even though these compositions have been stored for a period of time. In an embodiment, the preserved activity of the antifungal composition may last for a period of storage of over 90 days, 120 days, 150 days, 220 days or 260 days.

The antifungal compositions can be prepared in a ready-to-use formulation or commercialized in the form of kit so that the end-user can prepare the solution just before application. A method for the preparation of the antifungal composition comprises the step of mixing a garlic extract (such as a dehydrated garlic extract) with a chitosan or salt thereof. The garlic extract and the chitosan or its salt can be combined either in their liquid form or in a solid form (e.g. a powder form). Consequently, the resulting antifungal composition can be in a liquid form (such as an aqueous solution) or in a solid form (e.g. a powder form). When the antifungal composition is in a powder form, it must be combined with an aqueous solution or water before it is applied onto plants, parts thereof or product thereof.

When the compositions of the invention are combined with an aqueous solution or water, the composition of the garlic extract and the chitosan or salt thereof can be a more effective antifungal agent than if an equivalent amount of the garlic extract or the chitosan or salt thereof was used alone in aqueous solution. The composition of the garlic extract and the chitosan or salt thereof is more effective for controlling powdery mildew than if an equivalent amount of the garlic extract or the chitosan or salt thereof was used alone in aqueous solution.

It is also another object of the present invention to provide a method for controlling powdery mildew disease which would be easy to perform at a low cost. It is also another object of the present invention to provide a method for efficiently controlling powdery mildew disease, by means of one application per week.

The antifungal composition described herein can be used to inhibit the growth of a pathogen or controlling powdery mildew in a plant, parts thereof or products thereof. Consequently, there is provided a method for inhibiting growth of a pathogen in a plant, part thereof or product thereof and a method for controlling powdery mildew disease. The methods comprise the step of contacting the antifungal composition described herein with the plant, part thereof or product thereof. The antifungal composition can be produced by the methods described herein. The methods provide inhibition of the growth of various pathogens such as fungi (e.g. from the *Botrytis* sp. (e.g. *Botrytis cinerea),* from the *Fusarium* sp. (e.g *Fusarium oxysporum, Fusarium sambucinum, Fusarium graminearum),* from the *Penicillium* sp. (e.g. *Penicillium digitatum),* from the *Phytophthora* sp. (e.g. *Phytophthora megasperma),* from the *Pythium* sp. (e.g. *Pythium aphanidermatum, Pythium ultimum),* from the *Rhizoctonia* sp. (e.g. *Rhizoctonia solani),* from the *Sclerotinia* sp. (e.g. *Sclerotinia sclerotiorum),* from the *Verticillium* sp. (e.g. *Verticillium dahliae),* a powdery mildew pathogen or a rust pathogen). The antifungal composition may contact the entire plant, only a part thereof (such as a leaf, a foliage or a root) or the product of said plant. An advantage of the antifungal composition described herein is that it possesses long-lasting antifungal effects and that daily application of the composition is not necessary to inhibit pathogen growth (except in very severe case of pathogen growth or plant disease). The antifungal composition may applied at various intervals depending on the severity of the powdery mildew disease or the amount of pathogens present in the plant. In an embodiment, the antifungal composition may be applied only at least once every 5 days, 7 days, 14 days or 20 days. In addition, due to its low plant cytotoxicity, the number of applications of the antifungal compositions that can be applied to plants, parts thereof or products thereof can be higher than conventional plant pesticides.

The method for preparing the antifungal composition can also comprise drying a freshly obtained garlic extract and/or transforming the dehydrated garlic extract into a powder form. The powdered dehydrated garlic extract can optionally be inserted in a first container. Chitosan or its salt (preferably in powder form) can also be placed in the first container. The first container can be optionally sealed and stored for a predetermined period of time (e.g. for a period of over 260 days). In order to use the antifungal composition, the first container can be opened and its content can be mixed with water to obtain an aqueous solution. Alternatively, the chitosan and its salt can be placed in a second container that is optionally sealed and stored. In order to use the antifungal composition, the first and the second container can be opened and their content can be mixed. The contents of the first and second container can be mixed with water to reconstitute an aqueous solution, before or after the content of the two containers are mixed together.

One of the preferred method for controlling powdery mildew disease in a plant with the compositions of the present application, or for taking advantage of the antifungal activity of those compositions, consists in preparing the compositions of the present application and contacting the compositions with the plant, vegetable plant or vegetable thereof, or fruit plant or fruit thereof. More preferably, the compositions are contacted with a leaf or foliage of the plant, vegetable plant or vegetable thereof, or fruit plant or fruit thereof.

According to another aspect of the description, there is provided a method for enhancing an antifungal activity of a garlic extract, comprising the step of mixing said garlic extract with chitosan or a salt thereof. The expression "enhanced antifungal activity" as used herein refers to an antifungal activity that is substantially more effective than a regular or non-enhanced antifungal activity.

According to another aspect of the description there is provided a method for, enhancing a garlic extract activity toward controlling powdery mildew disease, said method comprising the step of mixing said garlic extract with chitosan or a salt thereof. The expression " enhanced activity for controlling powdery mildew disease " as used herein refers to an activity for controlling powdery mildew disease that is substantially more effective than a regular or non-enhanced activity for controlling powdery mildew disease.

According to another aspect of the description there is provided a method for extending the shelf life of a garlic extract, comprising the step of mixing said garlic extract with chitosan or a salt thereof. According to another aspect of the description, there is provided a method for preserving the activity of a garlic extract toward controlling powdery mildew disease, said method comprising the step of mixing said garlic extract with chitosan or a salt thereof. The expression "extended shelf life" as used herein refers to the property of the compositions to retain their antifungal activity or to retain their ability to control powdery mildew disease for a certain period during a storage. In a non-limitative manner, such an extended shelf life may last for a period of over 90 days, preferably for a period of over 120 days, more preferably for a period of over 150 days, more preferably for a period of over 220 days, and even more preferably for a period of over 260 days. When compared to a regular or non-extended shelf life, an extended shelf life will be maintained for a period of storage at least 25 times, and preferably 30 times, longer. The expressions "preserving the activity" and "preserved activity" as used herein refer to an activity that is substantially maintained during storage as opposed to a regular or non-preserved activity that is lost or reduced during storage. In a non-limitative manner, such a preserved activity lasts for a period of over 90 days, preferably for a period of over 120 days, and more preferably for a period of over 150 days, more preferably for a period of over 220 days, and even more preferably for a period of over 260 days. When compared to a regular or non-preserved activity for controlling powdery mildew disease, a preserved activity for controlling powdery mildew disease will be maintained for a period of storage at least 25 times, and preferably 30 times, longer.

The antifungal composition can be part of a kit for domestic or industrial use. In an embodiment, the kit comprises a garlic extract (e.g. in powder form) and a chitosan or a salt thereof (e.g. in powder form). The kit may optionally comprise instructions for mixing said garlic extract with said chitosan or salt thereof, thereby obtaining an antifungal composition. The instructions may also indicate to dilute the resulting powder in water to obtain an aqueous solution. The instructions may also indicate that the antifungal composition can be used for inhibiting the growth of a pathogen in a plant, part thereof or product thereof. The antifungal composition may inhibit the growth of a pathogen such as a fungus (e.g. from the *Botrytis* sp. (e.g. *Botrytis cinerea),* from the *Fusarium* sp. (e.g *Fusarium oxysporum, Fusarium sambucinum, Fusarium graminearum),* from the *Penicillium* sp. (e.g. *Penicillium digitatum),* from the *Phytophthora* sp. (e.g. *Phytophthora megasperma*), from the *Pythium* sp. (e.g. *Pythium aphanidermatum, Pythium ultimum),* from the *Rhizoctonia* sp. (e.g. *Rhizoctonia solani),* from the *Sclerotinia* sp. (e.g. *Sclerotinia sclerotiorum),* from the *Verticillium* sp. (e.g. *Verticillium dahliae),* a powdery mildew pathogen or a rust pathogen). The instructions may also set forth the use of the antifungal composition for controlling powdery mildew disease in a plant, part thereof or product thereof. In an embodiment, the garlic extract and the chitosan or salt thereof are being placed within a first container. The first container may be a pouch package made of a polymeric material. In another embodiment, the kit may also comprise a second container. In this particular embodiment, the garlic extract is placed with the first container and the chitosan or its salt is placed in a second container. The second container may be a pouch package made of a polymeric material. In an embodiment, the first and second container may be sealed under vacuum. The kit may effectively preserve the antifungal activity and/or the controlling activity against powdery mildew of the composition for a period of more than 260 days.

According to another aspect of the description, there is provided a method for increasing the yield of an infected plant, part thereof, or product thereof, comprising the step of contacting the antifungal solution described herein with the infected plant, part thereof or product thereof. The expression "infected plant" as used herein refers to a plant in which the growth of a fungal plant pathogen is occurring, or in which powdery mildew disease is occurring. The expressions "increasing the yield of an infected plant" and "yield increase of an infected plant" as used herein refer to the yield of a plant that is substantially increased in rate or in constancy in comparison with the yield of an infected plant that is not treated.

### EXAMPLE I -Preparation of Garlic Extract-based compositions

Compositions according to particular embodiments of the present invention have been prepared according to one the following general methods.
*Method 1 - Fresh garlic extracts.* Aqueous garlic extracts were prepared from fresh garlic bulbs by crushing surface-sterilized cloves. The pulp was suspended in sterile, distilled water containing 0.1% of vegetable cooking oil, filtered through sterile cheese cloth, and the resulting "stock" solution was diluted in distilled water to obtain final garlic concentrations ranging from 200 to 5 mg/ml. Chitosan lactate (obtained from Marinard Biotech, Riviere-au-Renard, Quebec, Canada) was resuspended in sterile distilled water to obtain final concentrations ranging from 10-1 mg/ml. For application in the greenhouse, fresh garlic extracts were mixed with chitosan lactate to obtain a final concentration of 50 mg/ml for garlic and 2 mg/ml for chitosan. The pH of the suspension was adjusted to 5.2-5.8.
*Method 2* - *Dehydrated garlic extracts.* Peeled or unpeeled garlic cloves were cut into halves and dehydrated prior to being ground to a fine powder and stored in vacuum-sealed bags at room temperature. For foliar application, the garlic powder was mixed with an appropriate amount of chitosan lactate and dissolved in tap water before use. The pH of the suspension was adjusted to 5.2-5.8.

Table 1 indicates the various compositions prepared and used in the following examples.

| **Table 1. Content of the various compositions tested** | |
|---|---|
| **compositions** | **Content** |
| composition 1 | - dehydrated garlic extract (50 mg/mL) - chitosan lactate (2mg/mL) - vegetable oil (0.01% v/v) - water |
| composition 2 | - dehydrated garlic extract (50 mg/mL) - vegetable oil (0.01% v/v) - water |
| composition 3 | - chitosan lactate (2mg/mL) - vegetable oil (0.01 % v/v) - water |
| composition 4 | - vegetable oil (0.01 % v/v) - water |
| composition 5 | - fresh garlic extract in liquid solution (200 mg/mL) - water - vegetable oil (0.01% v/v) |
| composition 6 | - fresh garlic extract in liquid solution (50 mg/mL) - chitosan lactate (2mg/mL) - water |

| **compositions** | **Content** |
|---|---|
| | - vegetable oil (0.01% v/v) |
| composition 7 | - dehydrated garlic extract (50 mg/mL) - water - vegetable oil (0.01% v/v) |
| composition 8 | - liquid garlic extract (60 % v/v) prepared from a 500 mg/mL solution, which is then diluted 1/50 v/v - water |
| composition 9 | - cinnamon oil (5 % v/v), diluted (1/50 v/v) - water |
| composition 10 | - cotton oil (25 % v/v), diluted (1/50 v/v) - water |
| composition 11 | - liquid garlic extract (60 % v/v) prepared from a 500 mg/mL solution, which is then diluted 1/50 v/v - cinnamon oil (5 % v/v), diluted (1/50 v/v) - water |
| composition 12 | - liquid garlic extract (60 % v/v) prepared from a 500 mg/mL solution, which is then diluted 1/50 (v/v) - cotton oil (25 % vlv), diluted (1/50 v/v) - water |
| composition 13 | - mineral oil (30% v/v), diluted (1/50 v/v) |
| composition 14 | - chitosan lactate (2 mg/mL) - red thyme oil (0.05 % v/v) - water |
| composition 15 | - chitosan lactate (2 mg/mL) - cinnamon oil (0.05 % v/v) - water |
| composition 16 | - dehydrated garlic extract (12.5 mg/mL) - chitosan lactate (2mg/mL) - vegetable oil (0.01% v/v) - water |
| composition 17 | - dehydrated garlic extract (25 mg/mL) - chitosan lactate (2mg/mL) - vegetable oil (0.01 % v/v) - water |
| composition 18 | - dehydrated garlic extract (100 mg/mL) - chitosan lactate (2mg/mL) - vegetable oil (0.01 % v/v) - water |

### EXAMPLE II - Determination of the antifungal properties of the antifungal compositions

In order to demonstrate the synergistic effect of garlic extracts and chitosan, several experiments were performed. Compositions 1 to 4 (Table 1) were applied by foliar spray 7 to 10 days after the inoculation of cucumber plants with powdery mildew spores (infected with 3-4 x 10⁴ spores/mL of *Podosphaera xanthii* (syn. *Sphaerotheca fuliginea)).* The following results were obtained and are summarized in Table 2.

| Table 2. Percentage of reduction of cucumber powdery mildew over different periods | | | | |
|---|---|---|---|---|
| of time upon treatment with compositions 1 to 4 | | | | |
| **compositions** | **3 days** | **6 days** | **8 days** | **12 days** |
| composition 1 | 100 % | 100% | 100% | 95% |
| composition 2 | 100 % | 80% | 65% | 65% |
| composition 3 | 30% | 30% | 45% | 45% |
| composition 4 | 10% | 0% | 0% | 0% |

Each composition was sprayed once on cucumber leaves and disease assessment was performed after 3, 6, 8 and 12 days. At the beginning of the treatment, the leaf infection rate ranged from 80 to 100%. For each composition, the assay was repeated three times and the results illustrated in Table 2 represent the average of these three assays. It should be mentioned that the vegetable oil did not exert a significant effect on the powdery mildew pathogen (composition 4, table 2).

Composition 1, made of dehydrated garlic extracts and chitosan lactate, is clearly more effective for controlling cucumber powdery mildew than compositions 2 or 3, made respectively of dehydrated garlic extracts alone or chitosan lactate alone. Evidence is provided from these results that chitosan lactate alone is not efficient. However, when chitosan lactate was combined to garlic extracts, the biological activity of the garlic extracts was enhanced. Consequently, a user that would treat the plant with composition 2 would have, few days after the first spray application, to provide the leaves with a second spray application in order to have a sufficient control of the disease. However, in the case of composition 1, even 12 days after the first spray application, the disease is still controlled at 95 %. Composition 1 exhibits a significant long-lasting antifungal activity or a long-lasting activity for controlling powdery mildew disease as compared to the other compositions.

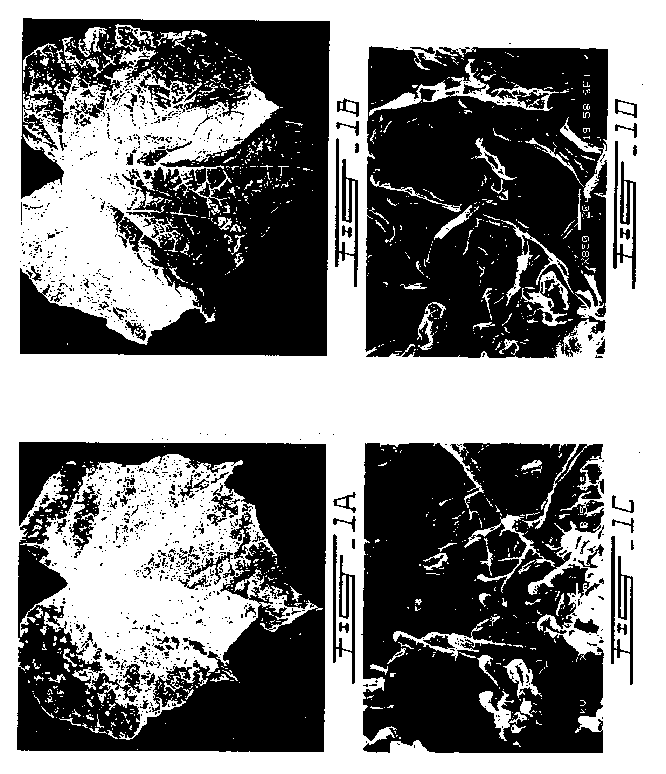

Figures 1A and 1B demonstrate clearly the effect of composition 1 on cucumber powdery mildew. 1C and 1D confirm that hyphae of the pathogen which were turgescent before treatment became completely collapsed after treatment with composition 1.

Therefore, the addition of a chitosan salt to garlic extracts greatly improves the antifungal activity of these extracts or their activity for controlling powdery mildew. Such a synergistic effect is particularly impressive after 8 days while composition 1 is considerably more efficient than composition 2.

The potential antifungal activity of compositions 1 to 4 was also tested against the plant pathogen, *Pythium ultimum,* grown *in vitro* on Potato Dextrose Agar (PDA) (see Table 3). Compositions 1-4 were introduced in the PDA at different concentrations as shown in table 3. Growth inhibition was measured 27 hours after inoculation of the pathogen.

| Table 3. Percentage of growth inhibition of *Pythium ultimum* with different dilutions of | | | | |
|---|---|---|---|---|
| compositions 1 to 4 | | | | |
| | **Concentration of the compositions in the PDA** | | | |
| **composition** | **12.50 mg/mL** | **6.25 mg/mL** | **3.13 mg/mL** | **1.56 mg/mL** |
| composition 1 | 100 % | 100 % | 100 % | 65 % |
| composition 2 | 100 % | 100 % | 75 % | 40 % |
| composition 3 | 40 % | 35 % | 30 % | 0 % |
| composition 4 | 0 % | 0 % | 0 % | 0 % |

The results presented in Table 3 confirm those previously shown in Table 2 concerning the synergistic effect of the composition made of chitosan lactate and dehydrated garlic extracts. It also supports the previous finding indicating that the vegetable oil has no antifungal activity and that chitosan lactate alone is not sufficient to generate a substantial growth inhibition.

Other assays have also been performed for clearly assessing that chitosan or a salt thereof can act as an efficient preservative for garlic extracts. Tables 2 and 3, show that chitosan or a salt thereof enhanced the antifungal activity of garlic extracts. Table 4 demonstrates that chitosan or a salt thereof preserves such an activity. Indeed, by using a garlic extract/chitosan or salt thereof composition, the antifungal properties of garlic are preserved or maintained over a prolonged period of time.

Compositions 5 to 7 (Table 1) have been prepared, stored either 0 or 7 days, and sprayed on cucumber leaves infected with powdery mildew at a rate of 90-100%. The efficiency of compositions 1 and 5 to 7 at controlling cucumber powdery mildew was assessed 7 days after leaf treatment. Table 4 summarizes the results obtained.

| Table 4. Percentage of reduction of infection of cucumber with powdery mildew upon | | |
|---|---|---|
| treatment with compositions 1, 5, 6 and 7 following different periods of storage. | | |
| **Compositions** | **Days of storage** | |
| | **0 day** | **7 days** |
| composition 5 | 90 % | 50 % |
| composition 6 | 100 % | 75 % |
| composition 7 | 100 % | 85 % |
| composition 1 | 100 % | 100 % |

Table 4 clearly shows that chitosan lactate (compositions 6 and 1) preserves the antifungal activity of garlic over time (compare composition 6 with composition 5; and composition 1 as compared to composition 7). Evidence is also provided that optimal preservation of the antifungal properties of garlic compounds is achieved by preparing solutions derived from dehydrated garlic extracts, compositions 1 and 5 to 7, when freshly prepared, have all a light beige color. However, after 24 hours, the aspect of compositions 5 and 7 change to yellow-green. This effect is even more amplified with composition 5 than with composition 7. By contrast, compositions 6 and 1 show very little changes after 24 hours. By 7 days after its preparation, composition 5 exhibits a deep green color, thus indicating that a strong oxidation of the garlic phenolic compounds occurred.

### EXAMPLE III - Comparison of (garlic extract / chitosan or a salt thereof) compositions with other garlic extract-based compositions or other compositions comprising chitosan salts

As previously indicated, some garlic-based compositions are described in U.S. Pat. No 6,231,865. Particular attention is paid to Samples 1 to 4 disclosed in column 2 of this document. Before further comparing the different samples of this document with compositions according to preferred embodiments of the present invention, the effect of sodium dodecyl sulfate itself (SDS), a chemical product present in Samples 1 to 4 of U.S. Pat. No 6,231,865, has been evaluated. An experiment similar to the one presented in example II has been carried out on powdery mildew-infected cucumber plants and an SDS-based aqueous solution. The results of this test are shown in Table 5.

| Table 5. Percentage of reduction of cucumber powdery mildew over different periods | | | | |
|---|---|---|---|---|
| of time when using a sodium dodecyl sulfate aqueous solution. | | | | |
| **Composition** | **3 days** | **6 days** | **8 days** | **12 days** |
| Sodium dodecyl sulfate | 100 % | 100% | 100% | 95% |
| (1/500 (w/v) in water) | | | | |

These results suggest that SDS is either a powerful antifungal product or a highly phytotoxic agent. Support to the concept that SDS was a toxic product came from the observation that phytoxicity developed in the days following the application of SDS. Examination of SDS-treated leaf samples by scanning electron microscopy revealed that the pathogen suffered also from serious damage. Since SDS is also known to be toxic for humans, it was voluntarily omitted in the compositions of US 6,231,865. Compositions 8 to 13 have thus been made accordingly to the teaching of US 6,231,865, with the exception that SDS was omitted (Table 1)

Compositions 1, and 8 to 13 (from Table 1) have then been applied to powdery mildew-infected cucumber plants (as previously described in Example II). Table 6 summarizes the results obtained.

| Table 6. Percentage of reduction of cucumber powdery mildew over different periods | | | | |
|---|---|---|---|---|
| of time after treatment with compositions 1, and 8 to 13. | | | | |
| **Compositions** | **3 days** | **6 days** | **8 days** | **12 days** |
| composition 1 | 100% | 100% | 100% | 90% |
| composition 8 | 80% | 50% | 30% | 25% |
| composition 9 | 90% | 50% | 40% | 25% |
| composition 10 | 0% | 0% | 0% | 0% |
| composition 11 | 100% | 60% | 30% | 30% |
| composition 12 | 50% | 40% | 25% | 20% |
| composition 13 | 0% | 0% | 0% | 0% |

Compositions 9 and 11 containing cinnamon oil were found to exert a certain antifungal activity. However, after 6 days, this activity decreased and a further spray application would have clearly been required. By contrast, the activity of composition 1 remained intact during the first week after the application, and thus plants would not have required an additional treatment. Composition 1 has obviously a superior antifungal activity as compared to compositions 8 to 13. Composition 1 clearly showed a significant long-lasting antifungal activity and/or a long-lasting activity for controlling powdery mildew as compared to the other compositions.

Compositions 1, 14 and 15 (Table 1) have been applied to powdery mildew-infected cucumber plants (as described in Example II).

| Table 7. Percentage of reduction of cucumber powdery mildew over different periods | | | | |
|---|---|---|---|---|
| of time with compositions 1, 14 and 15. | | | | |
| **compositions** | **3 days** | **6 days** | **8 days** | **12 days** |
| composition 1 | 100% | 100% | 100% | 100% |
| composition 14 | 90% | 80% | 60% | 40% |
| composition 15 | 100% | 70% | 40% | 25% |

Compositions 14 and 15, containing red thyme and cinnamon oil respectively, were quite effective after 3 days. However, this activity markedly decreased in the following days, suggesting that a further spray would have been required. Plants treated with composition 1 remained free of powdery mildew, even 12 days after the application, thus indicating that an additional spray was not necessary. Composition 1 appears to exert a much superior long-lasting antifungal activity than compositions 8 to 13.

All compositions listed in Table 7 were also tested for their ability to inhibit the growth of *Pythium ultimum* grown on PDA (as described in Example II). In each case, the pathogen was treated with compositions 1, 14 and 15, and the results were observed 27 and 47 hours after inoculation of the dishes (Table 8).

| Table 8. Percentage of growth inhibition of *Pythium ultimum* with compositions 1, 14 | | |
|---|---|---|
| and 15, over various periods of time. | | |
| **compositions** | **27 hours** | **47 hours** |
| composition 1 | 100% | 95% |
| composition 14 | 90% | 85% |
| composition 15 | 65% | 60% |

Table 8 confirms the results previously presented in Table 7 concerning the synergistic effect of the composition made of chitosan lactate and garlic extracts. Composition 1 has also a prolonged effect as compared to that of the other compositions.

### EXAMPLE IV - Growth inhibition of various pathogens using a composition comprising a garlic extract and chitosan and or a salt thereof

Compositions 1 and 16 to 18 (Table 1) have been tested on various pathogens to assess their efficacy. Compositions 16, 17 and 18 are similar to composition 1, except that the concentration of garlic extracts differs. These assays were carried out in a way similar to those presented in Example II. Fungal pathogens grown on PDA were subjected to compositions 1, 16, 17, and 18 and the percentage of growth inhibition was established 4 days after inoculation of the fungi on PDA amended with each of the compositions (Table 13).

| Table 9. Percentage of growth inhibition of various pathogens with compositions 1, | | | | |
|---|---|---|---|---|
| 16, 17 and 18. | | | | |
| **Pathogen** | **composition 1** | **composition 16** | **composition 17** | **composition 18** |
| *Botrytis cinerea* | 18% | 25% | 43% | 100% |
| *Fusarium* | 48% | 70% | 100% | 100% |
| *oxysporum* f.sp. | | | | |
| *radicis-lycopersici* | | | | |
| (FORL) | | | | |
| *Penicillium* | 68% | 91% | 100% | 100% |
| *digitatum* | | | | |
| *Phytophthora* | 62% | 100% | 100% | 100% |
| *megasperma* | | | | |
| *Pythium* | 100% | 100% | 100% | 100% |
| *aphanidermatum* | | | | |
| *Pythium ultimum* | 100% | 100% | 100% | 100% |
| *Rhizoctonia solani* | 69% | 85% | 100% | 100% |

Table 9 shows that compositions 1, 16, 17 and 18 are efficient on a wide range of pathogens with, however, a stronger activity against the oomycetes, *Pythium* and *Phytophthora* sp.

### EXAMPLE V - Treatment of powdery mildew-infected tomato and strawberry plants with the garlic extract / chitosan lactate composition

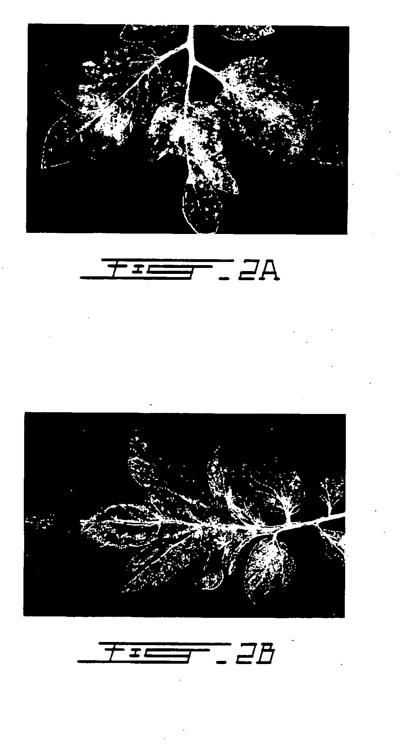

Powdery mildew infected-tomato plants were treated with composition 1 (Table 1), in a way similar to that presented above for cucumber plants (refer to Example II). Figures 2A and 2B, illustrate clearly the remarkable effect of composition 1 on tomato powdery mildew.

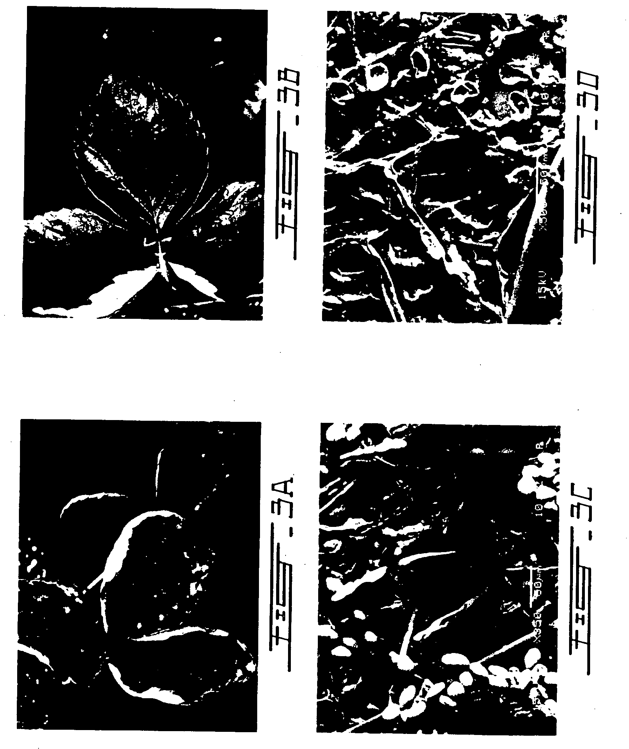

Similarly, powdery mildew-infected strawberry plants were treated with composition 1 (Table 1) in a way similar to that presented for cucumber plants (refer to Example II). The results obtained are illustrated in Figures 3A and 3B, thus demonstrating that composition 1 is also highly efficient at controlling strawberry powdery mildew, a devastating disease. Figures 3C and 3D provide evidence at the microscopic level that the fungus at the leaf surface is destroyed.

### EXAMPLE VI - Effect of the composition dehydrated garlic extracts-chitosan lactate in controlling cucumber powdery mildew in a semi-commercial greenhouse

Although the previous examples clearly indicated that the composition dehydrated garlic extracts-chitosan lactate was highly effective in fighting powdery mildew, further practical utilizations of the formulation required large-scale experiments under commercial growing conditions. A greenhouse trial was performed on cucumber plants and the effect of the dehydrated garlic-based composition on cucumber powdery mildew was compared to that of either a composition made of liquid garlic extracts and chitosan lactate or the chemical fungicide NOVA™.

Surface-sterilized cucumber seeds (cv. Corona) were sown in rock wool cubes and fertilized daily with a standard nutrient solution. Seedlings were grown for 4 wk on a greenhouse bench at 22-24°C with a 16 h photoperiod and transplanted into rook wool in 13.5 L plastic bags perforated on the upper face in order to insert two rock wool cubes per bag.-Plants at the 12-18 leaf stage were inoculated by gently applying a spore suspension of *P. xanthi* (3 x 10⁵ spores/ml) along the main vein of leaves 5 and 7. At the onset of powdery mildew sporulation (after 7-10 days), plants were sprayed with each composition or with the fungicide while controls were sprayed with water. The treatments were arranged in a randomized complete block design with 12 plants per treatment and three replications. Treatments with the garlic compositions were repeated once a week while treatment with NOVA™ were made at two-week intervals, in accordance with the manufacturer's recommendations.

Disease severity was assessed three and six days after each treatment and evaluated as percentage of leaf area covered by powdery mildew. The assessments were made on a "low" leaf level (about leaf 8), a "middle" leaf level (about leaf 15) and a "high" leaf level (about leaf 28) on three leaves for each level. For each treatment, cucumber yield was evaluated on 8 plants. Results obtained on week 12 after harvest on 8 plants of the "middle" level are summarized in table 10. Single degrees of freedom orthogonal contrasts were used to compare effects of NOVA™ vs. composition 1 (dehydrated garlic extracts-chitosan lactate) vs. composition 6 (fresh, liquid garlic extracts-chitosan lactate). The effects of control vs. NOVA™ and the two compositions were also compared in the same manner. Data were analyzed by analysis of variance using the SAS procedure (SAS Inst., Inc., Cary, NC). The LSMEANS option was used to generate the probability of difference between all the variables in the study.

| Table 10. Total number of fruits, class 1; total fruit yield, class 1; average fruit weight, | | | | |
|---|---|---|---|---|
| class 1; and percentage of leaf area covered by powdery mildew on 8 plants of long | | | | |
| English cucumber, cv. Corona , 12 weeks after harvest. | | | | |
| **Treatment** | **Yield** | | | **Disease incidence after 12 weeks (% of infection)** |
| | **Total number of fruits, class 1 (cucumbers/m²)** | **Av class 1 (kg/m²)** | **erage fruit Total fruit yield, Weight, class 1 (g)** | |
| **NOVA™** | 22 ± 5 | 10.2 ± 3.0 | 457 ± 27 | 0.20 ± 0.07 |
| **Composition 1** | 21 ± 5 | 9.6 ± 2.9 | 449 ± 32 | 0.03 ± 0.02 |
| **Composition 6** | 22 ± 2 | 9.3 ± 0.8 | 450 ± 8 | 0.13 ± 0.07 |
| **Control** | 11 ± 3 | 3.6 ± 1.0 | 338 ± 6 | 53.75 ± 4.17 |

These results demonstrate that: there is a significant difference between the control and the other treatments (p = 0.0121) for all variables analyzed; and that there is no significant difference between the three treatments for all variables analyzed. Evidence is also provided that the two compositions and the fungicide NOVA™ reduced significantly the incidence of powdery mildew by nearly 100%. However, repeated applications of NOVA™ resulted in some phytotoxicity as judged by the appearance of necrotic areas on the leaves which were abnormally curled. Application of both garlic-based compositions did not affect the plant physiology and morphology (data not shown). Therefore, the compositions were as effective as the synthetic fungicide without the associated phytotoxicity.

### EXAMPLE VII - Effect of the composition dehydrated garlic extracts-chitosan lactate in controlling tomato powdery mildew in a semi-commercial greenhouse

Surface-sterilized tomato seeds (cv. Trust) were sown in rock wool cubes and fertilized daily with a standard nutrient solution. Seedlings were grown for 4 wk on a greenhouse bench at 21-24°C with a 16 h photoperiod and transplanted (at the early flower stage) into rook wool in 13.5 L plastic bags perforated on the upper face in order to insert two rock wool cubes per bag. Plants at the 12-15 leaf.stage were inoculated by rubbing a piece of tomato leaf heavily infected by *Oidium neolycopersici* onto leaf 7. At the onset of the first powdery mildew colonies (after 7-10 days), plants were sprayed with each formulation or with sulphur (the currently applied fungicide in tomato greenhouses) while controls were sprayed with water. The treatments were arranged in a randomized complete block design with 6 plants per treatment and three replications. All treatments with the garlic compositions were repeated once a week.

The treatments included: 1) Dehydrated garlic extracts-chitosan lactate (composition 1); 2) Dehydrated garlic extracts alone (composition 2); 3) chitosan lactate alone (composition 3); 4) sulphur; 5) water; and 6) water + vegetable oil (composition 4).

Disease severity was assessed three and six days after each treatment and evaluated as percentage of leaf area covered by powdery mildew. The assessments were made on a "low" leaf level (about leaf 3), a "middle" leaf level (leaves 6 and 9) and a "high" leaf level (about leaf 12) on three leaves for each level. For each treatment, cucumber yield was evaluated on 3 plants per replication. Results obtained on week 18 after harvest on 8 plants of the "middle" level are summarized in table 11. Data were analyzed by analysis of variance using the SAS procedure (SAS Inst., Inc., Cary, NC). The LSMEANS option was used to generate the probability of difference between all the variables in the study. Single degrees of freedom orthogonal contrasts were used to compare effects of the composition vs. chitosan lactate alone, the composition vs. water or water and oil, and the composition vs. sulphur. It is of note that powdery mildew on tomato is much less aggressive than it is on cucumber. The disease occurs on the leaves as well-delimited spots which do not enlarge in a way similar to what can be observed with cucumber.

Our results indicate that the composition garlic-chitosan lactate, garlic extract alone and sulphur reduced significantly the incidence of powdery mildew in tomato by nearly 100%. By contrast, the effect of chitosan lactate alone was much lower with about 25 % of disease control. The composition appeared slightly better than the garlic extracts alone. In terms of fruit yield, the best results were obtained with the composition garlic-chitosan lactate, followed by garlic extracts alone and by sulphur. Surprisingly, chitosan lactate alone, which did not efficiently control the disease, did not affect substantially total fruit yield and total number of fruits (Table 11).

| Table 11. Total number of fruits, class 1; total fruit yield, class 1; average fruit weight, | | | | |
|---|---|---|---|---|
| class 1; and percentage of leaf area covered by powdery mildew on 3 plants of tomato, | | | | |
| cv. Trust, 14 weeks after harvest. | | | | |
| **Treatment** | **Yield** | | | **Disease incidence after 14 weeks (% of infection)** |
| | **Total fruit yield, class 1 (Kg/m²)** | **Total number of fruits, class 1 (tomatos/m²)** | **Average fruit Average fruit weight**, **class 1 (g)** | |
| ***Sulphur*** | 16.3 ± 1.1 | 100 ± 5.0 | 164 ± 3 | 0.28 ± 0.08 |
| ***Composition*** | 19.0 ± 1.1 | 111 ± 6.6 | 182 ± 2 | 0.30 ± 0.09 |
| ***Garlic extracts*** | 18.7 ± 1.3 | 107 ± 5.2 | 168 ± 6 | 0.34 ± 0.03 |
| ***Chitosan alone*** | 16.1 ± 1.2 | 100 ± 3.3 | 180 ± 6 | 1.20 ± 0.09 |
| **Water** | 11.8 ± 0.4 | 71 ± 1.6 | 166 ± 2 | 1.71±0.12 |
| **Water** + **oil** | 12.4 ± 0.5 | 79 ± 0.4 | 157 ± 5 | 1.60 ± 0.15 |

### EXAMPLE VIII - Effect of the garlic-chitosan composition in controlling rose powdery mildew

In order to confirm the beneficial effect of the garlic-based composition on rose powdery mildew, further experiments were conducted in a commercial greenhouse at Rose Drummond, Drummondville, Québec. Powdery mildew-infected rose plants (one row per treatment, 100 feet x 3 feet) were treated with either the composition fresh (composition 6) or dehydrated (composition 1) garlic extracts-chitosan lactate or with MELTATOX™, a currently-applied fungicide at a concentration of 3 ml/L. Controls included plants treated with water + vegetable oil (0.25 %). Treatments were applied on days 0, 4, 11, and 19. Because of its possible phytotoxicity, MELTATOX™ was applied on days 0, 4, and 19 only, in accordance with the manufacturer's recommendations.

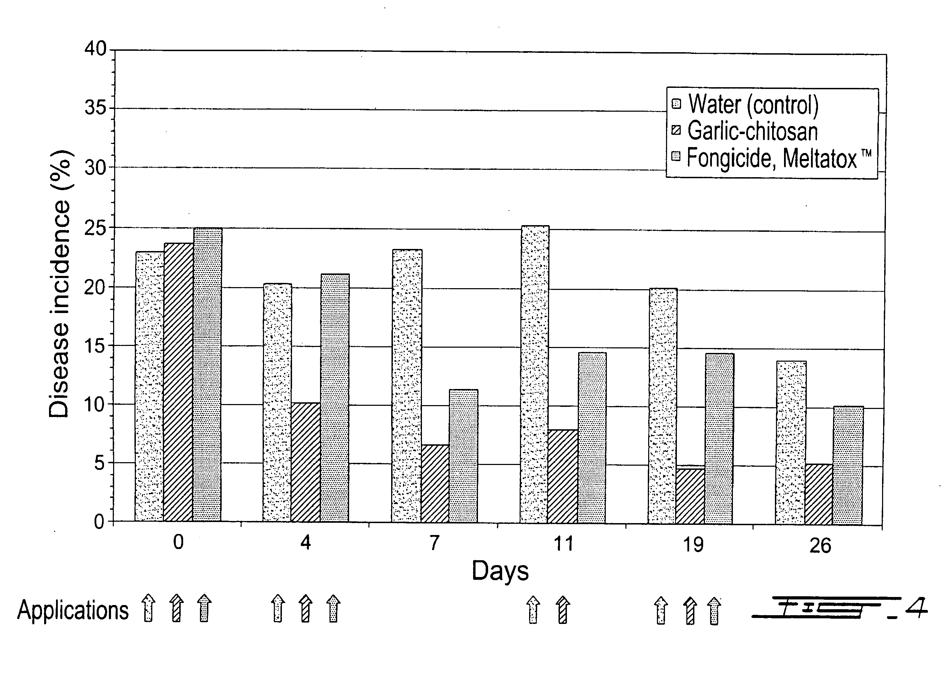

Disease severity was assessed on days 4, 7, 11, 19, and 26 on the first four leaves from the top of 20 plants per treatment (Fig. 4). On days 4, 11 and 19, disease severity was monitored just before the treatments. Results from this large-scale experiment provided additional evidence that the garlic-chitosan composition controlled efficiently rose powdery mildew even at a severe stage of the disease. Interestingly, the composition appeared more efficient than the fungicide MELTATOX™.

### EXAMPLE IX - Effect of the garlic-chitosan composition in controlling peony powdery mildew

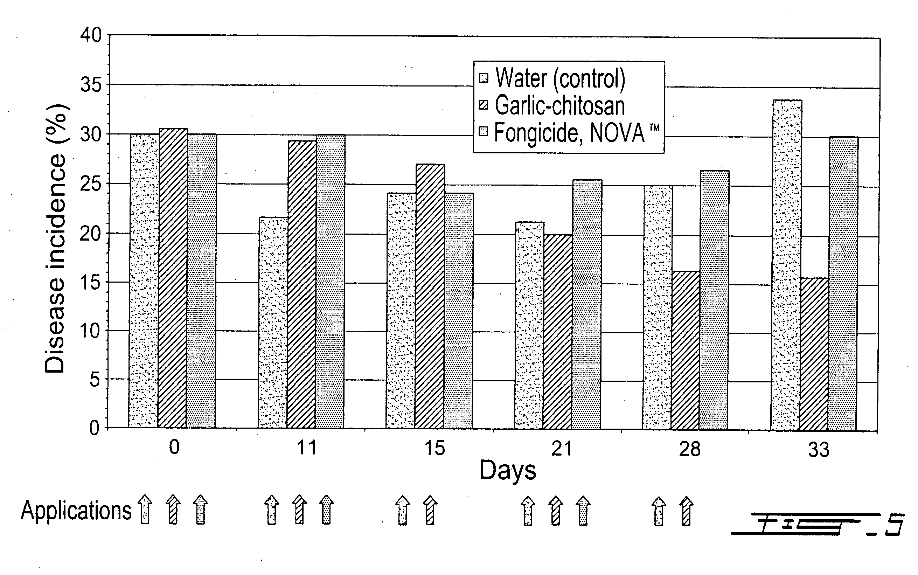

Naturally powdery mildew-infected peony plants (cvs. Colo, Moli and Bartzella), grown in a peat-amended organic substrate, were treated with either the composition fresh (composition 6) or dehydrated (composition 1) garlic extracts-chitosan lactate or with the fungicide NOVA™ at a concentration of 0.34g/L, in accordance with the manufacturer's recommendations. Controls included plants treated with water + vegetable oil (0.25 %). Treatments were applied on days 0, 11, 15, 21, and 28. The fungicide NOVA™ was applied on days 0, 11 and 21 according to manufacturer's indications. Disease severity was assessed just befor the treatments on days 4, 7, 11, 19, and 26 on three leaves of 2 plants per cultivar per treatment. Figure 5 represents the average for three cultivars.

Results from this experiment indicate that, in the case of peony, the garlic-based composition starts to be efficient after 3 applications. Evidence is also provided that the disease is not satisfactorily controlled by the fungicide NOVA™. Thus, the composition garlic-chitosan is a powerful alternative for the control of peony powdery mildew.

EXAMPLE X - Effect of the garlic-chitosan composition in controlling cucumber powdery mildew as compared to other bioproducts

This experiment was designed to determine whether or not the composition dehydrated garlic extracts-chitosan lactate was superior to commercially-available bioproducts for controlling powdery mildew. These bioproducts have been suggested to potentially have an impact on powdery mildew, although clear evidence was never provided. Cucumber plants, grown in a greenhouse maintained at 22/18°C (day/night) with a 16-h photoperiod, were inoculated at the 5-6 leaf stage with spores of *P. xanthi.* At the onset of powdery mildew sporulation (after 7-10 days ), plants were sprayed once a week with each bioproduct or with the composition dehydrated garlic extracts-chitosan lactate (composition 1), while controls were sprayed with water. The treatments were arranged in a randomized complete block design with 4 plants per treatment and three replications. Disease severity was assessed before each treatment on leave 2 and 4 and estimated as percentage of leaf area covered by powdery mildew.

The following bioproducts were compared to the composition dehydrated garlic extracts (50 mg/ml)-chitosan lactate (2 mg/ml) (composition 1) for their biological activity against cucumber powdery mildew.

MILSTOP™: Potassium bicarbonate (85 %) from BioWorks Inc., USA. It was used at a concentration of 2000L/ha = 2.8 g/L, in accordance with the manufacturer's recommendations.

FONGINEEM™: Potassium salts of fatty acids from neem oil (40 %) from Pronatex, Canada. It was used at a concentration of 25 ml/L, in accordance with the manufacturer's recommendations.

SILIFORCE™: Potassium silicate from Label Agro, Canada. It was used at a concentration of 1 ml/L, in accordance with the manufacturer's recommendations.

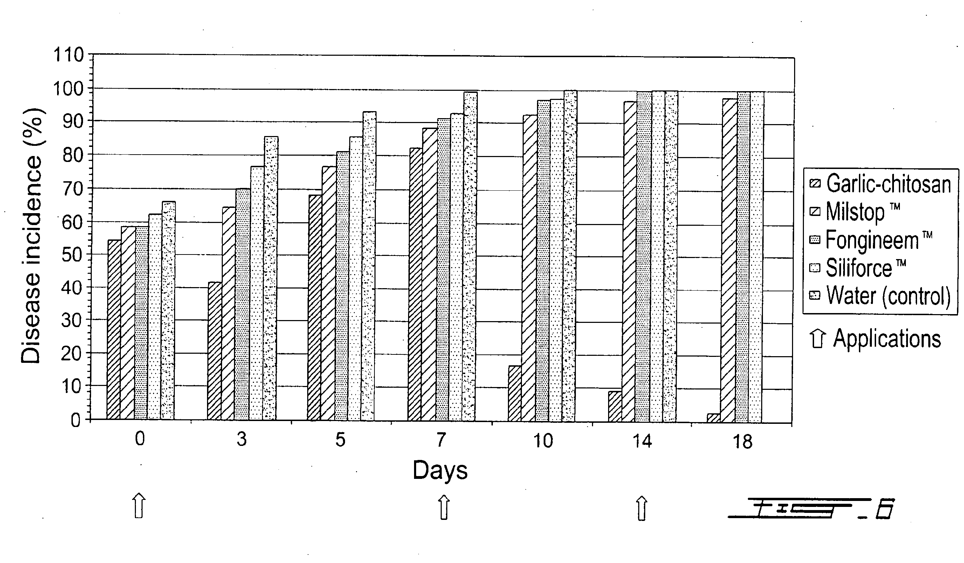

Figure 6 summarizes the results obtained over 18 days. Similar results were obtained for after over 260 days. The percentage of infection at the beginning of the experiment (day 0) averaged 60 %. Evidence is provided that, after two applications (Days 0 and 7) of the composition dehydrated garlic extracts (50 mg/ml)-chitosan lactate (2 mg/ml), the disease was significantly controlled since the percentage of leaf infection averaged 2 % by 18 days (Fig. 6). By contrast, MILSTOP™, FONGINEEM™ and SILIFORCE™ did not exert any significant effect even after three applications as evidenced by the propagation of the disease to nearly 100% of the leaf surface. Results from this experiment demonstrate that the dehydrated garlic extracts-chitosan lactate composition is a biological control approach that offers the best prospects as a reliable substitute of chemicals.

### EXAMPLE XI - Shelf-life of the composition dehydrated garlic extracts-chitosan lactate

Long term stability and storage quality of the composition were tested by using cucumber powdery mildew as a model. Concentrated dehydrated garlic extracts (as described in Example I), mixed with chitosan lactate at 1 or 2 mg/ml, were stored in vacuum-sealed bags either at room temperature or at 4°C. The effects of the dehydrated compositions were tested the day of the preparation (day 0) and after 10, 36, 68, 160 and 221 days on powdery mildew-infected cucumber plants. For each application, the dehydrated mixtures were dissolved in an appropriate amount of water and the pH adjusted at 5.3 with HCl 1 N. Infected cucumber plants were sprayed with each composition and the degree of control was assessed after 2-3 days. The tested formulations were:
Composition A: Dehydrated garlic extracts (50 mg/ml) + Chitosan lactate (1 mg/ml) at 4°C;
Composition B Dehydrated garlic extracts (50 mg/ml) + Chitosan lactate (1 mg/ml) at room temperature;
Composition C: Dehydrated garlic extracts (50 mg/ml) + Chitosan lactate (2 mg/ml) at 4°C;
Composition D: Dehydrated garlic extracts (50 mg/ml) + Chitosan lactate (2 mg/ml) at room temperature.

| Table 12. Shelf-life of compositions A-D. The degree of control of cucumber powdery | | | | | | |
|---|---|---|---|---|---|---|
| mildew was assessed 2-3 days after each treatment. | | | | | | |
| | Degree of control (%) | | | | | |
| Treatments | **Days of storage** | | | | | |
| | **0** | **10** | **36** | **68** | **160** | **221** |
| Composition A | 70 % | 75 % | 80 % | 78 % | 91 % | 95 % |
| Composition B | 75 % | 78 % | 80 % | 88 % | 85 % | 95 % |
| Composition C | 70 % | 75 % | 85 % | 68 % | 91 % | 95 % |
| Composition D | 75 % | 78 % | 80 % | 83 % | 88 % | 95 % |

Although the results may slightly vary from one application to another mainly due to the conditions that prevail in the greenhouse (sunny days vs. cloudy days, spring vs. autumn, temperature, etc.), the data obtained clearly demonstrate that storage either at room temperature or at 4°C does not affect the biological properties of the dehydrated mixtures. In addition, the chitosan lactate concentration (1 or 2 mg/ml) did not significantly modify the effect of the compositions. Similar results have been obtained when the compositions were stored 260 days prior to their useThus, the most important result from this experiment is that the dehydrated powders are still efficient at controlling cucumber powdery mildew after seven months of storage.

## Claims

1. A method for inhibiting, for a plant or a product of a plant, the growth of a fungus, said method comprising the step of contacting an antifungal composition comprising a garlic extract and a chitosan or a salt thereof with said plant or said product of a plant, thereby inhibiting the growth of said fungus.

2. A method for controlling, for a plant or a product of a plant, powdery mildew disease, said method comprising the step of contacting an antifungal composition comprising a garlic extract and a chitosan or a salt thereof with said plant or said product of a plant, thereby controlling powdery mildew disease in said plant.

3. The method of claim 1 or 2, wherein the pH of said antifungal composition is ranging from 5.0 to 6.0.

4. The method of claim 1 or 2, wherein said chitosan salt is selected from the group consisting of chitosan lactate, chitosan propionate, chitosan sorbate and chitosan gluconate.

5. The method of claim 4, wherein said chitosan salt is chitosan lactate.

6. The method of claim 1 or 2, wherein said chitosan is native chitosan.

7. The method of claim 1 or 2, wherein said antifungal composition is an aqueous solution.

8. The method of claim 7, wherein the concentration of said garlic extract in said antifungal composition is ranging from about 5 mg/mL to 95 mg/mL and the concentration of said chitosan or salt thereof in said antifungal composition is ranging from about 0.1 mg/mL to 6 mg/mL.

9. The method of claim 8, wherein the concentration of said garlic extract in said antifungal composition is ranging from about 10 mg/mL to 80 mg/mL and the concentration of said chitosan or salt thereof in said antifungal composition is ranging from about 0.2 mg/mL to 5 mg/mL.

10. The method of claim 1 or 2, wherein said antifungal composition is contacted with foliage of said plant.

11. The method of claim 10, wherein said plant is selected from the group consisting of a vegetable-producing plant, a fruit-producing plant and an ornamental plant.

12. The method of claim 1 or 2, wherein said product of said plant is selected from the group consisting of a vegetable and a fruit.

13. The method of claim 1, wherein said fungus is selected from the group consisting of *Botrytis cinerea, Fusarium oxysporum, Penicillium digitatum, Phytophthora megasperma, Pythium aphanidermatum, Pythium ultimum, Rhizoctonia solani, Sclerotinia sclerotiorum, Fusarium sambucinum, Fusarium graminearum, Verticillium dahliae,* a powdery mildew pathogen and a rust pathogen.

14. A method for preparing an antifungal composition, the method consisting essentially of the steps of mixing a garlic extract with a chitosan or a salt thereof and adjusting pH of said mixture to between 5.0 and 6.0, thereby obtaining said antifungal composition.

## Patentansprüche

1. Verfahren zum Hemmen des Wachstums eines Pilzes für eine Pflanze oder ein Produkt einer Pflanze, wobei das Verfahren den Schritt des Kontaktierens einer antimykotischen Zusammensetzung, die ein Knoblauchextrakt und ein Chitosan oder ein Salz davon umfasst, mit der Pflanze oder dem Produkt einer Pflanze umfasst, wodurch das Wachstum des Pilzes gehemmt wird.

2. Verfahren zum Kontrollieren einer Mehltauerkrankung für eine Pflanze oder ein Produkt einer Pflanze, wobei das Verfahren den Schritt des Kontaktierens einer antimykotischen Zusammensetzung, die ein Knoblauchextrakt und ein Chitosan oder ein Salz davon umfasst, mit der Pflanze oder dem Produkt einer Pflanze umfasst, wodurch die Mehltauerkrankung in der Pflanze kontrolliert wird.

3. Verfahren nach Anspruch 1 oder 2, wobei der pH-Wert der antimykotischen Zusammensetzung im Bereich von 5,0 bis 6,0 liegt.

4. Verfahren nach Anspruch 1 oder 2, wobei das Chitosansalz aus der Gruppe bestehend aus Chitosanlaktat, Chitosanpropionat, Chitosansorbat und Chitosangluconat ausgewählt ist.

5. Verfahren nach Anspruch 4, wobei es sich beim Chitosansalz um Chitosanlaktat handelt.

6. Verfahren nach Anspruch 1 oder 2, wobei es sich beim Chitosan um natives Chitosan handelt.

7. Verfahren nach Anspruch 1 oder 2, wobei es sich bei der antimykotischen Zusammensetzung um eine wässrige Lösung handelt.

8. Verfahren nach Anspruch 7, wobei die Konzentration des Knoblauchextrakts in der antimykotischen Zusammensetzung im Bereich von ungefähr 5 mg/ml bis 95 mg/ml liegt, und wobei die Konzentration des Chitosans oder Salzes davon in der antimykotischen Zusammensetzung im Bereich von ungefähr 0,1 mg/ml bis 6 mg/ml liegt.

9. Verfahren nach Anspruch 8, wobei die Konzentration des Knoblauchextrakts in der antimykotischen Zusammensetzung im Bereich von ungefähr 10 mg/ml bis 80 mg/ml liegt, und wobei die Konzentration des Chitosans oder Salzes davon in der antimykotischen Zusammensetzung im Bereich von ungefähr 0,2 mg/ml bis 5 mg/ml liegt.

10. Verfahren nach Anspruch 1 oder 2, wobei die antimykotische Zusammensetzung mit Blattwerk der Pflanze kontaktiert wird.

11. Verfahren nach Anspruch 10, wobei die Pflanze aus der Gruppe bestehend aus einer gemüseproduzierenden Pflanze, einer fruchtproduzierenden Pflanze und einer Zierpflanze ausgewählt ist.

12. Verfahren nach Anspruch 1 oder 2, wobei das Produkt der Pflanze aus der Gruppe bestehend aus einem Gemüse und einer Frucht ausgewählt ist.

13. Verfahren nach Anspruch 1, wobei der Pilz aus der Gruppe bestehend aus *Botrytis cinerea, Fusarium oxysporum, Penicillium digitatum, Phytophthora megasperma, Pythium aphanidermatum, Pythium ultimum, Rhizoctonia solani, Sclerotinia sclerotiorum, Fusarium sambucinum, Fusarium graminearum, Verticillium dahliae,* einem Mehltaupathogen und einem Rostpathogen ausgewählt ist.

14. Verfahren zum Herstellen einer antimykotischen Zusammensetzung, wobei das Verfahren im Wesentlichen die Schritte des Mischens eines Knoblauchextrakts mit einem Chitosan oder Salz davon und das Einstellen des pH-Werts der Mischung auf zwischen 5,0 und 6,0 umfasst, wodurch die antimykotische Zusammensetzung erhalten wird.

## Revendications

1. Procédé pour inhiber, chez une plante ou un produit d'une plante, la croissance d'un champignon, ledit procédé comprenant l'étape consistant à mettre en contact une composition antifongique comprenant un extrait d'ail et un chitosane ou un sel de celui-ci avec ladite plante ou ledit produit d'une plante, ce qui inhibe ainsi la croissance dudit champignon.

2. Procédé pour contrôler, chez une plante ou un produit d'une plante, le mildiou poudreux, ledit procédé comprenant l'étape consistant à mettre en contact une composition antifongique comprenant un extrait d'ail et un chitosane ou un sel de celui-ci avec ladite plante ou ledit produit d'une plante, ce qui contrôle ainsi la croissance du mildiou poudreux chez ladite plante.

3. Procédé selon la revendication 1 ou 2, dans lequel le pH de ladite composition antifongique est compris entre 5,0 et 6,0.

4. Procédé selon la revendication 1 ou 2, dans lequel ledit sel de chitosane est sélectionné dans le groupe consistant en du lactate de chitosane, du propionate de chitosane, du sorbate de chitosane et du gluconate de chitosane.

5. Procédé selon la revendication 4, dans lequel ledit sel de chitosane est du lactate de chitosane.

6. Procédé selon la revendication 1 ou 2, dans lequel ledit chitosane est du chitosane natif.

7. Procédé selon la revendication 1 ou 2, dans lequel ladite composition antifongique est une solution aqueuse.

8. Procédé selon la revendication 7, dans lequel la concentration dudit extrait d'ail dans ladite composition antifongique est comprise entre environ 5 mg/ml et 95 mg/ml, et la concentration dudit chitosane ou sel de celui-ci dans ladite composition antifongique est comprise entre environ 0,1 mg/ml et 6 mg/ml.

9. Procédé selon la revendication 8, dans lequel la concentration dudit extrait d'ail dans ladite composition antifongique est comprise entre environ 10 mg/ml et 80 mg/ml, et la concentration dudit chitosane ou sel de celui-ci dans ladite composition antifongique est comprise entre environ 0,2 mg/ml et 5 mg/ml.

10. Procédé selon la revendication 1 ou 2, dans lequel ladite composition antifongique est mise en contact avec le feuillage de ladite plante.

11. Procédé selon la revendication 10, dans lequel ladite plante est sélectionnée dans le groupe consistant en une plante produisant des légumes, une plante produisant des fruits et une plante ornementale.

12. Procédé selon la revendication 1 ou 2, dans lequel ledit produit de ladite plante est sélectionné dans le groupe consistant en un légume et un fruit.

13. Procédé selon la revendication 1, dans lequel ledit champignon est sélectionné dans le groupe consistant en *Botrytis cinerea, Fusarium oxysporum, Penicillium digitatum, Phytophthora megasperma, Pythium aphanidermatum, Pythium ultimum, Rhizoctonia solani, Sclerotinia sclerotiorum, Fusarium sambucinum, Fusarium graminearum, Verticillium dahliae,* un agent pathogène du mildiou poudreux et un agent pathogène de la rouille.

14. Procédé pour préparer une composition antifongique, le procédé comprenant essentiellement les étapes consistant à mélanger un extrait d'ail avec un chitosane ou un sel de celui-ci, et à ajuster le pH dudit mélange entre 5,0 et 6,0, ce qui permet ainsi d'obtenir ladite composition antifongique.
